(19) Europäisches Patentamt
European Patent Office
Office européen des brevets

(11) EP 1 208 082 B1

(12) EUROPÄISCHE PATENTSCHRIFT

(45) Veröffentlichungstag und Bekanntmachung des Hinweises auf die Patenterteilung:
**07.04.2004 Patentblatt 2004/15**

(21) Anmeldenummer: **00951530.5**

(22) Anmeldetag: **23.08.2000**

(51) Int Cl.⁷: **C07C 263/10**

(86) Internationale Anmeldenummer:
**PCT/EP2000/008221**

(87) Internationale Veröffentlichungsnummer:
**WO 2001/017951 (15.03.2001 Gazette 2001/11)**

(54) **VERBESSERTES VERFAHREN ZUR HERSTELLUNG VON MONO- UND OLIGOISOCYANATEN**

IMPROVED METHOD FOR PRODUCING MONOISOCYANATES AND OLIGOISOCYANATES

PROCEDE AMELIORE DE PREPARATION DE MONO- ET OLIGO-ISOCYANATES

(84) Benannte Vertragsstaaten:
**AT BE CH CY DE DK ES FI FR GB GR IE IT LI LU MC NL PT SE**

(30) Priorität: **04.09.1999 DE 19942299**

(43) Veröffentlichungstag der Anmeldung:
**29.05.2002 Patentblatt 2002/22**

(73) Patentinhaber: **BASF AKTIENGESELLSCHAFT**
**67056 Ludwigshafen (DE)**

(72) Erfinder:
- **STAMM, Armin**
  **D-55128 Mainz (DE)**
- **KNEUPER, Heinz-Josef**
  **D-67150 Niederkirchen (DE)**
- **THIL, Lucien**
  **D-67117 Limburgerhof (DE)**
- **HENKELMANN, Jochem**
  **D-68165 Mannheim (DE)**

(56) Entgegenhaltungen:
**US-A- 3 371 114** **US-A- 3 440 268**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von Monound Oligoisocyanaten durch Umsetzung von primären Aminen mit Phosgen in Gegenwart eines Katalysators.

Isocyanate sind großtechnische Produkte mit einer Vielzahl von Anwendungen auf dem Gebiet der Polyurethan-Kunststoffe. Bestimmte Isocyanate finden aber auch Anwendung bei der Herstellung pharmazeutischer Wirkstoffe.

Die Synthese von Isocyanaten durch Umsetzung von Aminen mit Phosgen ist seit langen bekannt. Prinzipiell werden zwei Verfahren in der Literatur beschrieben, von denen das eine bei Normaldruck und das andere bei erhöhtem Druck durchgeführt wird. Die Phosgenierung unter erhöhtem Druck ist nachteilig, da sie zur Beherrschung des erhöhten Sicherheitsrisikos, der Freisetzung von Phosgen, einen stark erhöhten apparativ technischen Aufwand erfordert.

Für Sulfonylisocyanate ist aus der US 3,371,114 und der US 3,484,466 ein Herstellungsverfahren bei Normaldruck bekannt, bei dem eine Lösung eines Sulfonylamids und eines Isocyanats als Katalysator in einem inerten Lösungsmittel mit Phosgen umgesetzt wird. Dabei wird intermediär der entsprechende Sulfonylharnstoff gebildet, welcher mit Phosgen zum gewünschten Sulfonylisocyanat reagiert.

Alkyl- und Arylisocyanate werden üblicherweise nach dem beispielsweise in Houben-Weyl, Methoden der organischen Chemie, 4. Auflage, Band E4, Seiten 741-751, Georg Thieme Verlag Stuttgart, 1983, beschriebenen Phosgenierungsverfahren in zwei Phasen bei Normaldruck aus den entsprechenden Aminen hergestellt. In der ersten Phase, der Kaltphosgenierung, wird das Amin mit einem Phosgenüberschuß in stark verdünnter Lösung und bei tiefen Temperaturen zum entsprechenden Carbaminsäurechlorid umgesetzt, aus dem in der zweiten Phase bei erhöhter Temperatur, der Heißphosgenierung, das Isocyanat entsteht. Aliphatische und cycloaliphatische primäre Amine sind dabei aufgrund ihrer gegenüber aromatischen Aminen erhöhten Basizität schlechter zu phosgenieren und führen zu einem vermehrten Anfall an Nebenprodukten. Nachteilig an diesen Verfahren ist neben der Phosgenierung in zwei Phasen, die Bildung einer intermediären Feststoffsuspension aus schwerlöslichem Carbaminsäurechlorid und Aminhydrochlorid, die wiederum eine erhöhte Verdünnung des Reaktionsmediums erforderlich macht, um Ablagerungen und Verstopfungen von Anlagenteilen zu verhindern. Aufgrund des vorhandenen Feststoffanfalls kann dieses Verfahren bei Normaldruck nicht kontinuierlich geführt werden. Weiterhin entsteht symmetrisch N,N'-substituierter Harnstoff als Nebenprodukt, dessen Bildung nur um den Preis drastisch verringerter Raum-Zeit-Ausbeuten unterdrückt werden kann.

Aliphatische und cycloaliphatische Amine werden häufig in Form ihrer Salze in die Kalt/Heißphosgenierung eingesetzt. Diese Salze sind jedoch im Reaktionsmedium schwer löslich, so daß zusätzliche Reaktionsstufen und sehr lange Reaktionszeiten erforderlich werden.

Weiterhin ist aus der GB 1 114 085, der US 3 492 331 und H. Ulrich, Chemistry & Technology of Isocyanates, Wiley & Sons, 1996, Seiten 328-330, bekannt, die Umsetzung primärer Amine mit Phosgen durch den Zusatz von Katalysatoren wie Dimethylformamid, Phenyltetramethylguanidin, 2,4,6-Trimethylpyridin oder Carbodiimidazol zu optimieren. Diese Katalysatoren müssen zum Teil in äquimolaren Mengen eingesetzt werden und bilden unter den Reaktionsbedingungen schwer lösliche Salze.

Die Aufgabe der vorliegenden Erfindung ist es, ein für aliphatische, cycloaliphatische, araliphatische und aromatische primäre Amine anwendbares Verfahren zur Herstellung der entsprechenden Mono- und Oligoisocyanate mit Phosgen bereitzustellen, das sowohl kontinuierlich als auch diskontinuierlich bei Normaldruck geführt werden kann, die vorstehenden Nachteile nicht aufweist und in guten Ausbeuten und hohen Selektivitäten die entsprechenden Mono- und Oligoisocyanate zur Verfügung stellt.

Die Aufgabe wird gelöst durch ein Verfahren zur Herstellung von aliphatischen, cycloaliphatischen, araliphatischen und aromatischen Mono- und Oligoisocyanaten durch Phosgenierung der entsprechenden primären Amine bei Normaldruck, bei dem man

a) eine katalytische Menge eines Monoisocyanats (Isocyanat a) in einem inerten Lösungsmittel mit Phosgen vorlegt,

b) das primäre Amin zusetzt und

c) das erhaltene Reaktionsgemisch mit Phosgen umsetzt.

Die dem Verfahren zugrundeliegende Bruttogleichung ist im folgenden Schema 1 wiedergegeben.

Schema 1:

$$R_1\text{-}NH_2 + COCl_2 \xrightarrow[-2HCl]{R_2\text{-}N=C=O} R_1\text{-}N=C=O$$

Das erfindungsgemäße Verfahren hat den Vorteil, daß es für eine Vielzahl von Aminen anwendbar ist. Die Phosgenierung wird erfindungsgemäß unter Vermeidung einer Trennung in Kalt- und Heißphosgenierung in einem schmalen Temperaturintervall und bei Normaldruck durchgeführt, wobei die intermediäre Bildung von schwerlöslichen Suspensionen vermieden wird. Das gewünschte Isocyanat wird dabei bei vollständigem Umsatz des Amins in hohen Ausbeuten und hoher Selektivität in deutlich verkürzten Reaktionszeiten gebildet, ohne daß symmetrisch substituierter N,N'-Harnstoff aus dem Amin als Nebenprodukt gebildet wird. Da eine Harnstoffbildung aus dem Amin nicht beobachtet wird, ist es durch das erfindungsgemäße Verfahren möglich, die Konzentration des Amins in der Reaktionslösung und damit die Raum-Zeit Ausbeuten deutlich zu erhöhen. Zudem ist es vorteilhaft, daß das erfindungsgemäße Verfahren aufgrund des ausbleibenden Feststoffanfalls sowohl in diskontinuierlicher als auch in kontinuierlicher Fahrweise durchgeführt werden kann.

Durch das erfindungsgemäße Verfahren sind aliphatische, cycloaliphatische, araliphatische und aromatische Mono- und Oligoisocyanate der Formel I

$$R_1 - N = C = O \qquad \text{(I)}$$

erhältlich. Der Rest $R_1$ in Formel I entspricht dem Rest $R_1$ in Formel IV der in dem erfindungsgemäßen Verfahren eingesetzten Amine, die später diskutiert werden, und auf die hier verwiesen wird. Nach dem erfindungsgemäßen Verfahren sind bevorzugt Mono- und Diisocyanate herstellbar. In der Praxis von weniger großer Bedeutung jedoch prinzipiell herstellbar, sind Isocyanate mit 3 und mehr Isocyanatgruppen.

Als alleiniger Katalysator wird ein Monoisocyanat der allgemeinen Formel II (Isocyanat a)

$$R_2 - N = C = O \qquad \text{(II)}$$

oder dessen Gemische eingesetzt, worin $R_2$ für aliphatische, cycloaliphatische, aromatische oder araliphatische Reste steht. Diese können durch Heteroatome substituiert sein, bzw. ihre Kohlenstoffketten können durch Heteroatome wie Sauerstoff und Schwefel unterbrochen sein. Der Rest $R_2$ muß jedoch gegenüber Phosgen inert sein, so daß NH-, OH- und SH-Gruppen tragende Reste ausgeschlossen sind. Die aliphatischen Reste können beliebig verzweigt oder unverzweigt, gesättigt oder ungesättigt sein. Sie enthalten 3 bis 30 Kohlenstoffatome, vorzugsweise 3 bis 10 Kohlenstoffatome. Beispiele für aliphatische Reste sind Methyl, Ethyl, Propyl, n-Butyl, iso-Butyl, sec-Butyl.

Als cycloaliphatische Reste kommen solche in Betracht, die 3 bis 20 Kohlenstoffatome, vorzugsweise 3 bis 10 Kohlenstoffatome, aufweisen, wie zum Beispiel Cyclopentyl und Cyclohexyl.

Die aromatischen Reste können unsubstituiert oder beliebig mit Alkyl-, Arylsubstituenten oder Heteroatomen substituiert sein. Bevorzugt sind die aromatischen Reste, die mono- oder disubstituiert sind. Beispiele für aromatische Reste sind Phenyl-, Chlorphenyl, o-, m- und p-Tolyl.

Als araliphatische Reste sind Reste mit 7 bis 12 Kohlenstoffatomen, wie zum Beispiel Benzyl geeignet, bevorzugt ist jedoch ein Rest der Formel III

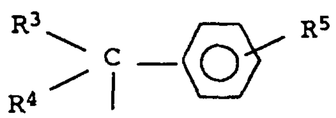

(III),

worin $R_3$ und $R_4$ gleich oder verschieden sein können und für Wasserstoff oder einen aliphatischen, cycloaliphatischen, araliphatischen oder aromatischen Rest stehen können, wobei diese durch Heteroatome substituiert sein können, bzw.

ihre Kohlenstoffketten können durch Heteroatome wie Sauerstoff und Schwefel unterbrochen sein. Die Reste $R_3$ und $R_4$ müssen jedoch gegenüber Phosgen inert sein, so daß NH-, OH- und SH-Gruppen tragende Reste ausgeschlossen sind. Die aliphatischen Reste können beliebig verzweigt oder unverzweigt, gesättigt oder ungesättigt sein. Sie enthalten 1 bis 30 Kohlenstoffatome, vorzugsweise 1 bis 10 Kohlenstoffatome. Beispiele für aliphatische Reste sind Methyl, Ethyl, Propyl, n-Butyl, iso-Butyl, sec-Butyl.

Als cycloaliphatische Reste kommen solche in Betracht, die 3 bis 20 Kohlenstoffatome, vorzugsweise 3 bis 10 Kohlenstoffatome, aufweisen, wie zum Beispiel Cyclopentyl und Cyclohexyl.

Die aromatischen Reste können unsubstituiert oder beliebig mit Alkyl-, Arylsubstituenten oder Heteroatomen substituiert sein. Bevorzugt sind die aromatischen Reste, die mono- oder disubstituiert sind. Beispiele für aromatische Reste sind Phenyl-, Chlorphenyl, o-, m- und p-Tolyl. $R^5$ kommt die Bedeutung Fluor, Chlor, Brom oder einer $C_1$- bis $C_4$-Alkylkette, welche gegebenenfalls durch ein Heteroatom, beispielsweise Sauerstoff oder Schwefel unterbrochen sein kann, zu. Bevorzugt ist das Monoisocyanat (Isocyanat a), ein lineares aliphatisches Alkylisocyanat, besonders bevorzugt ein lineares aliphatisches Alkylisocyanat mit 3 bis 10 Kohlenstoffatomen, wobei die Alkylkette gegebenenfalls verzweigt sein kann, insbesondere bevorzugt n-Butylisocyanat. Im Fall der Herstellung von Monoisocyanaten nach dem erfindungsgemäßen Verfahren ist es auch möglich, das als Produkt gewünschte Monoisocyanat in katalytischen Mengen als Isocyanat a zu verwenden. Es ist jedoch bevorzugt ein nicht mit dem Produkt (Isocyanat der Formel (I)) identisches Isocyanat a als Katalysator einzusetzen.

Das Isocyanat a wird im allgemeinen in einer katalytische Menge von 0,01 bis 25 mol-%, bevorzugt 0,5 bis 20 mol-%, besonders bevorzugt 1 bis 15 mol-%, bezogen auf das Amin, eingesetzt.

Im Schritt a) des erfindungsgemäßen Verfahrens wird das Isocyanat a in einem gegenüber Phosgen inerten Lösungsmittel vorgelegt. Bevorzugte Lösungsmittel sind Kohlenwasserstoffe. Besonders bevorzugt sind einfach oder mehrfach substituierte aromatische Kohlenwasserstoffe, wie Toluol, o-, m- oder p-Xylol, Ethylbenzol, Chlorbenzol oder 1,2-, 1,3- oder 1,4-Dichlorbenzol oder deren Gemische. Insbesondere bevorzugt sind Xylole, Chlorbenzol und Dichlorbenzole als Lösungsmittel.

Das erfindungsgemäße Verfahren wird bei Normaldruck und im allgemeinen bei einer über die drei Schritte des Verfahrens a), b) und c) weitgehend konstanten Reaktionstemperatur von 20 bis 200°C, bevorzugt 20 bis 150°C, insbesondere bevorzugt 50 bis 120°C durchgeführt.

Das in dem inerten Lösungsmittel gelöste Monoisocyanat (Isocyanat a) wird in Schritt a) des erfindungsgemäßen Verfahren auf die Reaktionstemperatur erwärmt und mit einem Überschuß an Phosgen, bezogen auf das Monoisocyanat vorgelegt. Vorzugsweise beträgt das Molverhältnis zwischen Phosgen und Isocyanat a 100 : 1 bis 5 : 1. Besonders bevorzugt wird Phosgen in einem Überschuß von 20 : 1 bis 5 : 1, bezogen auf das Isocyanat a, eingesetzt.

Das Phosgen kann in dem erfindungsgemäßen Verfahren in den Schritten a) und c) gasförmig oder in kondensierter Form eingesetzt werden, wobei es bevorzugt ist, das Phosgen gasförmig einzuleiten.

Als primäre Amine sind in dem erfindungsgemäßen Verfahren Amine der allgemeinen Formel IV

$$R_1 - NH_2 \qquad (IV)$$

geeignet, die nach literaturbekannten Verfahren oder als Handelsprodukt erhältlich sind.

$R_1$ steht für aliphatische, cycloaliphatische, aromatische oder araliphatische Reste. Diese können durch Heteroatome substituiert sein, bzw. ihre Kohlenstoffketten können durch Heteroatome wie Sauerstoff und Schwefel unterbrochen sein. OH- und SH-Gruppen enthaltende Reste sind ausgenommen. Eine Substitution durch Aminogruppen ist hingegen möglich und führt zu Di- oder Oligoisocyanaten, wobei Diisocyanate bevorzugt sind. Die aliphatischen Reste können beliebig verzweigt oder unverzweigt, gesättigt oder ungesättigt sein. Sie enthalten 3 bis 30 Kohlenstoffatome, vorzugsweise 3 bis 10 Kohlenstoffatome. Beispiele für aliphatische Reste sind Propyl, Butyl, Pentyl und Hexyl. Als cycloaliphatische Reste kommen solche mit 3 bis 20 Kohlenstoffatomen in Betracht, vorzugsweise solche mit 3 bis 10 Kohlenstoffatomen, wie Cyclopentyl und Cyclohexyl.

Die aromatischen Reste können 4 bis 10 Kohlenstoffatome aufweisen, heteroaromatische Reste sein und unsubstituiert oder beliebig mit Alkyl-, Arylsubstituenten oder Heteroatomen substituiert sein. Bevorzugt sind die aromatischen Reste mono- oder disubstituiert. Beispiele für aromatische Reste sind Phenyl und Chlorphenyl, o-, m-, p-Tolyl, Furfuryl, Thiophenyl und Pyrrolyl.

Als araliphatische Reste sind Reste mit 7 bis 12 Kohlenstoffatomen wie zum Beispiel Benzyl geeignet, bevorzugt ist jedoch ein Rest der Formel V

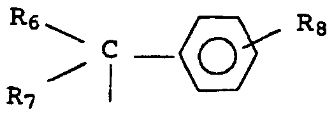

(V),

worin $R_6$ und $R_7$ gleich oder verschieden sein können und für Wasserstoff oder einen aliphatischen, cycloaliphatischen oder aromatischen Rest stehen können, wobei diese durch Heteroatome substituiert sein können, bzw. ihre Kohlenstoffketten können durch Heteroatome wie Sauerstoff und Schwefel unterbrochen sein. Die Reste $R_6$ und $R_7$ müssen jedoch gegenüber Phosgen inert sein, so daß NH-, OH- und SH-Gruppen tragende Reste ausgeschlossen sind. Die aliphatischen Reste können beliebig verzweigt oder unverzweigt, gesättigt oder ungesättigt sein. Sie enthalten 1 bis 30 Kohlenstoffatome, vorzugsweise 1 bis 10 Kohlenstoffatome. Beispiele für aliphatische Reste sind Methyl, Ethyl, Propyl, n-Butyl, iso-Butyl, sec-Butyl.

Als cycloaliphatische Reste kommen solche in Betracht, die 3 bis 20 Kohlenstoffatome, vorzugsweise 3 bis 10 Kohlenstoffatome, aufweisen, wie zum Beispiel Cyclopentyl und Cyclohexyl.

Die aromatischen Reste können unsubstituiert oder beliebig mit Alkyl-, Arylsubstituenten oder Heteroatomen substituiert sein. Bevorzugt sind die aromatischen Reste, die mono- oder disubstituiert sind. Beispiele für aromatische Reste sind Phenyl-, Chlorphenyl, o-, m- und p-Tolyl. $R_8$ kommt die Bedeutung Halogenatom (F, Cl, Br) oder einer $C_1$- bis $C_4$-Alkylkette, welche gegebenenfalls durch ein Heteroatom, beispielsweise Sauerstoff oder Schwefel, unterbrochen sein kann, zu.

Als primäre Amine der Formel IV können auch Amine in enantiomerenreiner, optisch aktiver Form oder Gemische der Enantiomeren eingesetzt werden. Das erfindungsgemäße Verfahren führt während der Reaktion und der Aufarbeitung vorteilhafterweise einem lediglich geringen Anteil an Racemisierung, der im allgemeinen unter 2 % liegt.

Bevorzugt werden in dem erfindungsgemäßen Verfahren cycloaliphatische, araliphatische und aromatische Amine eingesetzt, insbesondere solche, die in großtechnischen Prozessen weiterverarbeitet werden wie zum Beispiel Hexamethylendiaminen, Cyclohexylamin, Isophorondiamin, Toluylendiamin, Anilin sowie optisch aktive Amine wie zum Beispiel R-(+)- und S(-)-Phenylethylamin.

Das primäre Amin wird in Schritt b) des erfindungsgemäßen Verfahrens der mit Phosgen gesättigten Lösung des Monoisocyanats (Isocyanats a) bevorzugt in einem inerten Lösungsmittel zugesetzt. Dies kann beispielsweise in Gegenwart eines Inertgasstromes, beispielsweise Stickstoff, erfolgen. Bevorzugte Lösungsmittel sind die in Schritt a) des erfindungsgemäßen Verfahrens verwendeten Lösungsmittel. Besonders bevorzugt wird das gleiche Lösungsmittel wie in Schritt a) verwendet. Sodann wird das erhaltene Reaktionsgemisch in Schritt c) des erfindungsgemäßen Verfahrens mit Phosgen umgesetzt.

Das Molverhältnis zwischen primärem Amin und der Gesamtmenge der in Schritt a) und c) eingeleiteten Menge an Phosgen beträgt 1 : 10 bis 1 : 1, besonders bevorzugt wird Phosgen in einer Menge von 120 bis 180 mol-%, bezogen auf das Amin eingeleitet.

Das erfindungsgemäße Verfahren kann prinzipiell sowohl kontinuierlich als auch diskontinuierlich durchgeführt werden, wobei eine kontinuierliche Verfahrensführung bevorzugt ist. Das Verfahren kann in einer beliebigen, für eine Umsetzung mit Phosgen geeigneten Apparatur erfolgen. Beispielsweise wird eine Phosgenierungsapparatur eingesetzt, die einen aufgesetzten Kohlensäurekühler oder eine nachgeschaltete Intensivkühlungskaskade zur Phosgenkondensation umfaßt.

Für die bevorzugte kontinuierliche Fahrweise wird eine Phosgenierungsapparatur verwendet, die einen Haupt- und Nachreaktor, eine Strippkolonne und einen dieser nachgeschalteten Kondensationskühler umfaßt. Im Fall der kontinuierlichen Fahrweise wird bevorzugt ein Gemisch aus dem gewünschten Isocyanat der Formel I, den katalytischen Mengen des Isocyanats der Formel II (Isocyanat a) und Phosgen in dem inerten Lösungsmittel vorgelegt.

Nach einer Nachreaktion wird überschüssiges Phosgen und Lösungsmittel im allgemeinen bei Temperaturen von 30 bis 80°C, bevorzugt 40°C bis 60°C herausgestrippt, in dem Kondensationskühler kondensiert und zusammen in den Nachreaktor zurückgeführt.

Der Reaktionsaustrag wird mit dem Fachmann bekannten Methoden aufgearbeitet. Vorzugsweise wird das gewünschte Isocyanat der Formel I durch fraktionierte Destillation isoliert. Das als Katalysator eingesetzte Isocyanat a wird dabei bevorzugt durch Destillation zurückgewonnen und kann bei weiteren Ansätzen wieder verwendet werden. Die gewünschten Mono- und Oligoisocyanate können dabei in Ausbeuten von im allgemeinen 60 bis 95 %, bezogen auf das eingesetzte primäre Amin, erhalten werden.

Die folgenden Beispiele erläutern die Erfindung zusätzlich.

Beispiel 1: R-(+)-Phenylethylisocyanat

In einer Phosgenierungsapparatur mit nachgeschalteter Intensivkühlungskaskade wurden 500 g Chlorbenzol und 4,8 g n-Butylisocyanat (0,1 mol) vorgelegt. Bei Raumtemperatur wurden insgesamt innerhalb von 60 Minuten 50 g Phosgen eingegast. Danach wurde der Ansatz auf 72°C erhitzt, wobei sich ein kräftiger Phosgenrückfluß einstellte. Bei 72-82°C wurden insgesamt 121 g (1 mol) R-(+)-Phenylethylamin, gelöst in 200 g Chlorbenzol, innerhalb von 2 h zugetropft und gleichzeitig 100 g Phosgen eingeleitet. Nach einer Nachreaktionszeit von 3 h bei 80-85°C, wurde überschüssiges Phosgen mit Stickstoff bei 40°C ausgestrippt. Der Rohaustrag enthielt laut gaschromatographischer Bestimmung 86 % R-(+)-Phenylethylisocyanat (Lösungsmittel herausgerechnet) und wurde fraktioniert destilliert. Bei 0,5 mbar und 63°C wurden 122,1 g R-(+)-phenylethylisocyanat isoliert (83 % der Theorie).

Beispiel 2: R-(+)-Phenylethylisocyanat

In einer Phosgenierungsapparatur mit nachgeschalteter Intensivkühlungskaskade wurden 2,4 g n-Butylisocyanat (0,05 mol) in 400 g Chlorbenzol vorgelegt. Bei Raumtemperatur wurden insgesamt 50 g Phosgen eingegast. Danach wurde der Ansatz auf 79°C erhitzt, wobei sich ein kräftiger Phosgenrückfluß einstellt. Bei 79-82°C wurden insgesamt 121 g (1 mol) R-(+)-Phenylethylamin, gelöst in 200 g Chlorbenzol innerhalb von 1 h 40 min zugetropft und gleichzeitig 100 g Phosgen eingeleitet. Nach einer Nachreaktionszeit von 3,5 h bei 82-87°C, wurde überschüssiges Phosgen mit Stickstoff bei 40°C ausgestrippt. Der Rohaustrag enthielt laut gaschromatographischer Bestimmung 89 % R-(+)-Phenylethylisocyanat (Lösungsmittel herausgerechnet). Durch fraktionierte Destillation wurden bei 0,4 mbar und 66°C 121 g R-(+)-Phenylethylisocyanat isoliert (82 % der Theorie).

Beispiel 3: Kontinuierliche Fahrweise

Es wird eine Phosgenierungsapparatur, die einen Haupt-, einen Nachreaktor, eine Strippkolonne und eine nachgeschaltete Intensivkühlungskaskade umfaßt, verwendet.

In den Hauptreaktor, der mit einer Lösung aus 73,6 g R-(+)-Phenylethylisocyanat (0,5 mol; hergestellt nach den Beispielen 1-2) und 2,48 g (0,025 mol) n-Butylisocyanat in 250 g Chlorbenzol gefüllt war, wurden parallel innerhalb von 12 h kontinuierlich 164,7 g/h (entsprechend 0,26 mol/h R-(+)-Phenylethylamin) einer vorgemischten Lösung von 484 g (4 mol) R-(+)-Phenylethylamin mit einem Enantiomerenüberschuß von 97,2 % und 9,9 g (0,1 mol) n-Butylisocyanat in 2000 g Chlorbenzol und durchschnittlich 40,8 g/h Phosgen (entsprechend 0,41 mol/h) bei einer Temperatur von 70-75°C eingeleitet. Der Nachreaktor wurde innerhalb eines Temperaturbereiches von 80-85°C, die Strippkolonne, in der überschüssiges Phosgen kontinuierlich ausgestrippt wurde, auf eine konstante Temperatur von 50°C temperiert. Insgesamt wurden 2139 g Austrag erhalten. Der Gehalt an R-(+)Phenylethylisocyanat im Reaktionsaustrag wurde gaschromatographisch bestimmt, betrug 19,7 GC-Flächen-%, bzw. 90 Flächen-% (Lösungsmittel herausgerechnet). Durch fraktionierte Destillation wurden daraus 363 g R-(+)-Phenylethylisocyanat mit einer Reinheit > 98 % und einem Enantiomerenüberschuß von 95,7 % erhalten. Im Sumpf verblieben weitere 60 g mit einem Gehalt von 45 % R-(+)-Phenylethylisocyanat.

Beispiel 4: Isophorondiisocyanat

4,9 g n-Butylisocyanat (0,05 mol) und 111g (0,5 mol) Isophorondiisocyanat wurden in 300 g Chlorbenzol vorgelegt und auf 100°C erhitzt. Innerhalb von 15 Minuten wurden 25 g Phosgen eingegast, wobei Phosgen-Rückfluß einsetzte. Innerhalb von 2 h wurden weitere 91 g Phosgen und 85,2 g (0,5 mol), Isophorondiamin (3-(Aminomethyl)-3,5,5-trimethylcyclohexylamin), gelöst in 200 g Chlorbenzol, parallel bei 94-98°C zudosiert. Nach einer Nachreaktion von 4 h bei 111-113°C wurde überschüssiges Phosgen durch Strippung mit Stickstoff bei 40°C ausgetrieben. Der Rohaustrag von 645 g enthielt nach Gaschromatogramm 23 Flächen-% Isophorondiisocyanat. Durch fraktionierte Destillation im Vakuum (1,5 mbar, 120-128°C) wurden insgesamt 168 g Isophorondiisocyanat isoliert.

Beispiel 5: Cyclohexylisocyanat

In einer Phosgenierungsapparatur mit nachgeschalteter Intensivkühlungskaskade wurden 2,4 g (0,025 mol) n-Butylisocyanat in 500 g Chlorbenzol vorgelegt. Es wurden 40 g Phosgen (0,4 mol) eingeleitet und die Lösung auf 78°C erhitzt. Innerhalb von 140 Minuten wurden 99,2 g (1 mol) Cyclohexylamin (gelöst in 200 g Chlorbenzol) zugetropft und parallel weitere 110 g (1,1 mol) Phosgen gasförmig eingeleitet. Die Reaktionstemperatur wurde dabei zwischen 78 und 83°C gehalten. Nach einer Nachreaktionszeit von 1 h bei 87°C wurde das überschüssige Phosgen mit Stickstoff ausgestrippt. Der Austrag von 802 g enthielt nach Gaschromatogramm 11,9 Flächen-% Cyclohexylisocyanat. Fraktionierte Destillation im Vakuum lieferte 98 g (78 % d.T.) Cyclohexylisocyanat.

Beispiel 6: Phenylisocyanat

In einer Phosgenierungsapparatur mit nachgeschalteter Intensivkühlungskaskade wurden 2,4 g (0,025 mol) n-Butylisocyanat in 500 g Chlorbenzol vorgelegt. Es wurden 40 g Phosgen (0,4 mol) eingeleitet und die Lösung auf 81°C erhitzt. Innerhalb von 140 Minuten wurden 93,1 g (1 mol) Anilin (gelöst in 200 g Chlorbenzol) zugetropft und parallel weitere 110 g (1,1 mol) Phosgen gasförmig eingeleitet. Die Reaktionstemperatur wurde dabei zwischen 81 und 86°C gehalten. Nach einer Nachreaktionszeit von 1 h bei 88°C wurde das überschüssige Phosgen mit Stickstoff ausgestrippt. Der Austrag von 811 g enthielt nach Gaschromatogramm 12,4 Flächen-% Phenylisocyanat. Fraktionierte Destillation im Vakuum lieferte 91 g (76 % d.T.) Phenylisocyanat.

Vergleichsbeispiel 1

In einer Phosgenierungsapparatur mit nachgeschalteter Intensivkühlungskaskade wurden 4,8 g n-Butylisocyanat (0,05 mol) in 400 g Chlorbenzol vorgelegt. Bei Raumtemperatur wurden insgesamt 60,5 g R-(+)-Phenylethylamin (0,5 mol) innerhalb von 10 Minuten zugetropft. Danach wurde der Ansatz auf 70°C erhitzt. Bei 80-86°C wurden insgesamt innerhalb von 65 Minuten 61 g (0,61 mol) Phosgen eingeleitet. Nach erfolgtem Einleiten von 13 g Phosgen setzte Kristallbildung ein, nach Einleiten von 27 g Phosgen hatte sich eine dickflüssige Suspension gebildet, die sich nach Einleiten der gesamten Menge Phosgen nur langsam wieder auflöste. Nach einer Nachreaktion von 2 h 30 Minuten bei 80°C wurde das überschüssige Phosgen bei 40°C mit Stickstoff ausgestrippt. Der Reaktionsaustrag von 467 g enthielt nach gaschromatographischer Analyse 63,4 Flächen-% (ohne Lösungsmittel) R-(+)-Phenylethylisocyanat und 17,1 % höhersiedende Komponenten.

Das vergleichsbeispiel 1 zeigt im Vergleich zu den erfindungsgemäßen Beispielen 1 und 2 eine deutlich schlechtere Ausbeute und verringerte Selektivität. Zudem wird die Bildung einer Suspension beobachtet.

Vergleichsbeispiel 2: Kalt-Heiß-Phosgenierung

In einer Phosgenierungsapparatur mit nachgeschalteter Intensivkühlungskaskade wurden in 1000 g Chlorbenzol innerhalb von 130 Minuten bei -5 bis +5°C 330 g Phosgen einkondensiert.

Über 50 Minuten wurden 181,5 g R-Phenylethylamin (1,5 mol, gelöst in 750 g Chlorbenzol) zugetropft. Es entstand eine dicke Suspension, die gerade noch rührbar war. Nach vollständiger Aminzugabe wurde innerhalb von 1 h auf 59°C aufgeheizt. Es stellte sich Phosgenrückfluß ein. Der Ansatz wurde 90 Minuten bei 59-68°C gerührt, wobei die Lösung vollständig klar wurde. Es wurde weitere 3,5 h bei 68-86°C gerührt. Danach wurde innerhalb von 2,5 h unter Stickstoff auf 129°C erwärmt. Bei 125-130°C wurden weitere 30 g Phosgen eingeleitet und 2 h unter Rückfluß bei 126°C gerührt. Unter Kühlung auf 50°C wurde zunächst 3,5 h und dann weitere 4 h bei Raumtemperatur mit Stickstoff überschüssiges Phosgen ausgestrippt. Vom Reaktionsaustrag wurden zunächst bei 40 mbar und 43-44°C 1600 g Chlorbenzol abdestilliert. Der Rückstand wurde an einer 30 cm-Kolonne (gefüllt mit 5 mm Glasringen) fraktioniert destilliert. Die Hauptfraktion ging bei 7 mbar und 74-75°C über. Es wurden insgesamt 179 g (entspricht 81 % der Theorie) R-(+)-Phenylethylisocyanat mit einer Reinheit von 98,5 % isoliert.

Das Vergleichsbeispiel illustriert die Nachteile der üblichen Kalt-Heiß-Phosgenierung nämlich lange Reaktionszeiten, die Bildung einer im technischen Maßstab schwer handhabbaren Suspension sowie die niedrige Konzentration des Amins/des Isocyanates in der Lösung (Endkonzentration an Isocyanat ca. 11 Gew.-%). Das erfindungsgemäße Verfahren ermöglicht wie die Beispiele 1 und 2 zeigen deutlich höhere Konzentrationen (16-20 Gew.-% des Amins/Isocyanats in der Lösung). Bei zusätzlich niedrigeren Versuchsdauern bedeutet dies eine erhebliche Steigerung der erreichbaren Raum-Zeit-Ausbeute.

**Patentansprüche**

**1.** Verfahren zur Herstellung von aliphatischen, cycloaliphatischen, araliphatischen und aromatischen Mono- und Oligoisocyanaten durch Phosgenierung der entsprechenden primären Amine bei Normaldruck, **dadurch gekennzeichnet, daß** man

a) eine katalytische Menge eines Monoisocyanats (Isocyanat a) in einem inerten Lösungsmittel mit Phosgen vorlegt;

b) das primäre Amin zusetzt und

c) das erhaltene Reaktionsgemisch mit Phosgen umsetzt.

2. Verfahren nach Anspruch 1, **dadurch gekennzeichnet, daß** es bei 50 bis 120°C durchgeführt wird.

3. Verfahren nach Anspruch 1 oder 2, **dadurch gekennzeichnet, daß** die Umsetzung in o-, m-, p-Xylol, Chlorbenzol, Dichlorbenzolen oder deren Gemisch durchgeführt wird.

4. Verfahren nach einem der Ansprüche 1 bis 3, **dadurch gekennzeichnet, daß** das Isocyanat a in einer Menge von 0,01 bis 25 mol-%, bezogen auf das primäre Amin eingesetzt wird.

5. Verfahren nach einem der Ansprüche 1 bis 4, **dadurch gekennzeichnet, daß** als Isocyanat a ein lineares, aliphatisches $C_3$bis $C_{10}$-Monoalkylisocyanat eingesetzt wird.

6. Verfahren nach einem der Ansprüche 1 bis 5, **dadurch gekennzeichnet, daß** als primäres Amin ein aliphatisches $C_3$- bis $C_{30}$-Alkylamin, ein cycloaliphatisches $C_3$- bis $C_{20}$-Cycloalkylamin, ein araliphatisches Amin mit 7 bis 12 Kohlenstoffatomen oder ein $C_4$- bis $C_{10}$-Arylamin eingesetzt wird.

7. Verfahren nach einem der Ansprüche 1 bis 6, **dadurch gekennzeichnet, daß** als primäres Amin Hexamethylendiamin, Cyclohexylamin, Isophorondiamin, Toluylendiamin, Anilin, R-(+)-oder S-(-)-Phenylethylamin eingesetzt wird.

**Claims**

1. A process for the preparation of aliphatic, cycloaliphatic, araliphatic and aromatic mono- and oligoisocyanates by phosgenation of the corresponding primary amines at atmospheric pressure, which comprises

   a) introducing a catalytic amount of a monoisocyanate (isocyanate a) into an inert solvent together with phosgene;

   b) adding the primary amine, and

   c) reacting the resulting reaction mixture with phosgene.

2. A process as claimed in claim 1, which is carried out at from 50 to 120°C.

3. A process as claimed in claim 1 or 2, wherein the reaction is carried out in o-, m-, p-xylene, chlorobenzene, dichlorobenzenes or mixtures thereof.

4. A process as claimed in any of claims 1 to 3, wherein the isocyanate a is used in an amount of from 0.01 to 25 mol%, based on the primary amine.

5. A process as claimed in any of claims 1 to 4, wherein the isocyanate a used is a linear, aliphatic $C_3$- to $C_{10}$-monoalkyl isocyanate.

6. A process as claimed in any of claims 1 to 5, wherein the primary amine used is an aliphatic $C_3$- to $C_{30}$-alkylamine, a cycloaliphatic $C_3$- to $C_{20}$-cycloalkylamine, an araliphatic amine having from 7 to 12 carbon atoms or a $C_4$- to $C_{10}$-arylamine.

7. A process as claimed in any of claims 1 to 6, wherein the primary amine used is hexamethylenediamine, cyclohexylamine, isophoronediamine, toluylenediamine, aniline, R-(+)- or S-(-)-phenylethylamine.

**Revendications**

1. Procédé de préparation de mono- et d'oligoisocyanates aliphatiques, cycloaliphatiques, araliphatiques et aromatiques par phosgénation des amines primaires appropriées à pression normale, **caractérisé en ce que** :

   a) on dispose une quantité catalytique d'un monoisocyanate (isocyanate a) dans un solvant inerte avec le phosgène ;

b) on ajoute l'amine primaire, et
c) on fait réagir le mélange réactionnel obtenu avec le phosgène.

**2.** Procédé selon la revendication 1, **caractérisé en ce qu'**il est réalisé de 50 à 120°C.

**3.** Procédé selon la revendication 1 ou 2, **caractérisé en ce que** la réaction est réalisée dans le o-, le p- ou le m-xylène, le chlorobenzène, les dichlorobenzènes ou leurs mélanges.

**4.** Procédé selon l'une quelconque des revendications 1 à 3, **caractérisé en ce que** l'isocyanate a est mis en oeuvre en une quantité allant de 0,01 à 25% en poids, sur base de l'amine primaire.

**5.** Procédé selon l'une quelconque des revendications 1 à 4, **caractérisé en ce que** l'on met en oeuvre comme isocyanate a, un mono(alkyl en $C_3$ à $C_{10}$)isocyanate aliphatique, linéaire.

**6.** Procédé selon l'une quelconque des revendications 1 à 5, **caractérisé en ce que** l'on met en oeuvre comme amine primaire, une (alkyl en $C_3$ à $C_{30}$)amine aliphatique, une (cycloalkyl en $C_3$ à $C_{20}$)amine aliphatique, une amine araliphatique ayant 7 à 12 atomes de carbone ou une (aryl en $C_4$ à $C_{10}$)amine.

**7.** Procédé selon l'une quelconque des revendications 1 à 6, **caractérisé en ce que** l'on met en oeuvre comme amine primaire, l'hexaméthylènediamine, la cyclohexylamine, l'isophoronediamine, la toluylènediamine, l'aniline, la R-(+)- ou S-(-)-phényléthylamine.